**Europäisches Patentamt**

**European Patent Office**

**Office européen des brevets**

(19)

(11) Veröffentlichungsnummer: **0 206 982**
**B1**

(12) **EUROPÄISCHE PATENTSCHRIFT**

(45) Veröffentlichungstag der Patentschrift:
25.01.89

(51) Int. Cl.⁴: **A 61 M 11/00**, A 61 F 7/00

(21) Anmeldenummer: **86810198.1**

(22) Anmeldetag: **01.05.86**

(54) Vorrichtung zur Behandlung von Brandwunden oder Verbrühungen.

(30) Priorität: **17.05.85 CH 2123/85**

(43) Veröffentlichungstag der Anmeldung:
**30.12.86 Patentblatt 86/52**

(45) Bekanntmachung des Hinweises auf die Patenterteilung:
**25.01.89 Patentblatt 89/4**

(84) Benannte Vertragsstaaten:
**AT BE CH DE FR GB IT LI LU NL SE**

(56) Entgegenhaltungen:
**WO-A-84/04883**
**GB-A-1 104 214**
**GB-A-1 162 790**
**US-A-638 481**
**US-A-3 366 279**

(73) Patentinhaber: **IDC- Chemie AG, Schwerzistrasse 24, CH- 8807 Freienbach (CH)**

(72) Erfinder: **Mäder, Karl, Eichholzstrasse 41, CH- 8808 Pfäffikon (CH)**
Erfinder: **Schönenberger, Guido, Unterer Rebbergweg 138, CH- 4153 Reinach (CH)**
Erfinder: **Buzzi, Carlo A., Stauffacherstrasse 98, CH- 8004 Zürich (CH)**

(74) Vertreter: **White, William, Isler AG Patentanwalts- Bureau Walchestrasse 23, CH- 8006 Zürich (CH)**

EP 0 206 982 B1

## Beschreibung

Es wurde festgestellt, dass nach schweren Verbrennungen bei bereits stabilisiertem Zustand des Patienten häufig schwere Infektionen mit manchmal letalen Folgen auftreten. Dabei wurde ermittelt, dass durch Hitzeeinwirkung in der Haut entstehende giftige Substanzen (Verbrennungstoxine) die grundlegende Ursache für den tödlichen Verlauf der Verbrennungskrankheit ist. Diese giftigen, durch thermische Zersetzung von Eiweissen gebildeten Stoffe schwächen die Immunabwehr, so dass der Körper der nicht zu vermeidenden Einschwemmung von Bakterien durch die meist grossflächigen Wunden nicht mehr gewachsen ist.

Es wurde gefunden, dass eine sofortige Kühlung, d.h. innert 30 - 60 Sekunden, der Brandwunde mit kaltem Wasser in mehrfacher Hinsicht sehr günstig ist: Zunächst wird der Schmerz nachhaltig gemindert. Die Zersetzung von Eiweissen und Bildung von Verbrennungstoxinen wird stark reduziert oder gestoppt. Die Wunde verheilt wesentlich rascher und die Narbenbildung ist weniger störend.

Nun steht allerdings häufig kaltes Wasser als Erste-Hilfe-Massnahme nicht genügend rasch zur Verfügung. Der Erfindung liegt die Aufgabe zugrunde, diesem Mangel abzuhelfen. Diese Aufgabe wird durch die im Anspruch 1 definierte Erfindung gelöst.

Mit der Erfindung ist eine Vorrichtung geschaffen, die leicht greifbar überall aufgestellt werden kann, wo die Gefahr einer Verbrennung oder Verbrühung besteht. Durch die Trennung der Agentien in zwei Behältern kann die aufzutragende Flüssigkeit, vorzugsweise Wasser, keimfrei und mit langer Haltbarkeit bereitgestellt werden. Die durch das Kältemittel abgekühlte Flüssigkeit hat eine relativ hohe Kühlkapazität, so dass sie über einen hinreichenden Zeitraum die Brandwunde zu kühlen vermag. Dies im Gegensatz zu handelsüblichen Kältesprays, die sich rasch verflüchtigen und daher nur eine oberflächliche Abkühlung ermöglichen. Mit dem Kältespray kann vorzugsweise das Wasser nach dem Auftragen zu einer Eisschicht gefroren werden. Dies erhöht erheblich die Langzeitwirkung der Kühlung, weil Eis eine sehr hohe Kühlkapazität hat.

Der Anspruch 1 ist abgegrenzt gegenüber der U.S. Patentschrift 3 366 279. Diese Vorrichtung zeigt zwei Flüssigkeitsbehälter und ein gemeinsames Ventil, das wahlweise Flüssigkeit aus dem einen oder andern Behälter versprüht. Gedacht ist dabei insbesondere an Farbsprays, wobei z. B. zunächst eine Grundierung, anschliessend mit dem gleichen Gerät ein Überzugslack aufgetragen werden kann. Als medizinische Anwendung ist an das Auftragen zunächst eines antiseptischen Mittels, anschliessend eines Filmüberzuges gedacht.

Nachfolgend werden Ausführungsbeispiele der Erfindung anhand der Zeichnung erläutert. Darin zeigt:

Fig. 1 einen Axialschnitt einer ersten Ausführungsform, und
Fig. 2 eine Frontansicht einer zweiten Ausführungsform.

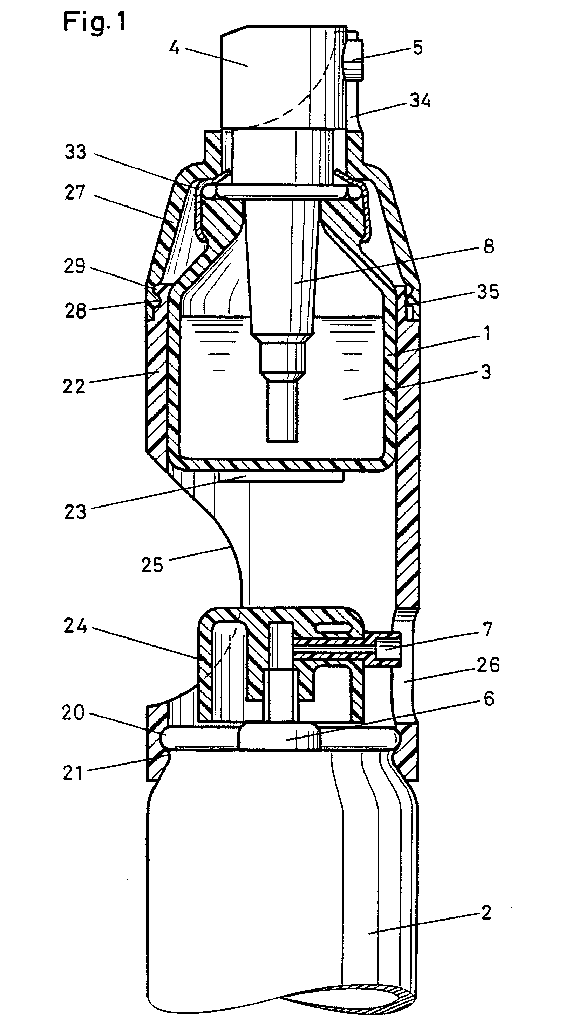

Im Ausführungsbeispiel nach Fig. 1 sind die beiden Behälter 1, 2 koaxial übereinander angeordnet. Der Behälter 1 enthält keimfreies Wasser 3 und ist wesentlich kleiner, als der ein Kältemittel, z. B. $C Cl_2 F_2$, enthaltende Behälter 2. Auf einen Bördelrand 20 des Behälters 2 ist mittels eines ringförmigen Vorsprungs 21 ein Gehäuse 22 aufgeschnappt, welches eine zylindrische Bohrung und einen axialen Anschlag 23 für die Aufnahme des Behälters 1 aufweist. Zur Betätigung eines Betätigungsknopfes 24 eines Ventils 6 des Behälters 2 ist im Gehäuse 22 ein Griffloch 25 ausgespart. Diametral gegenüberliegend dem Griffloch 25 hat das Gehäuse 22 ein Langloch 26, in welches die Sprühdüse 7 des Ventils 6 ragt. Der Behälter 1 ist im Gehäuse 22 durch eine aufgeschnappte Führungskappe 27 gehalten. Diese hintergreift mit einem ringförmigen Vorsprung 28 einen Vorsprung 29 des Gehäuses 22. Auf den Behälter 1 ist mittels einer aufgebördelten Krone 33 eine auf den Grund des Behälters 1 ragende Pumpe 8 luftdicht aufgesetzt. Der federbelastete Betätigungsknopf 4 der Pumpe 8 hat eine Sprühdüse 5, welche in einem axialen Schlitz 34 der Führungskappe 27 geführt ist. Damit der Schlitz 34 mit dem Langloch 26 fluchtet und somit die beiden Spühdüsen 5, 7 parallel gerichtet sind, hat die Führungskappe 27 einen Vorsprung 35, welcher in eine axiale Nut des Gehäuses 22 eingreift.

Das Wasser 3 im Behälter 1 bleibt wegen des luftdichten Abschlusses des Behälters 1 über lange Zeit keimfrei. Die Pumpe 8 gewährleistet auch nach jahrelanger Lagerung einen sicheren Betrieb der Vorrichtung. Statt der Pumpe 8 käme jedoch auch z. B. ein Blasenspeicher in Frage, bei welchem das Wasser durch eine Membran von einem Treibgas getrennt wäre.

Bei der Anwendung der beschriebenen Vorrichtung wird zunächst durch Druck auf den Betätigungsknopf 4 Wasser auf die Brandwunde gesprüht. Anschliessend wird dieser Wasserfilm durch Druck auf den Betätigungsknopf 24 mit einem Strahl des Kältemittels aus der Spühdüse 7 vereist, so dass die Brandwunde wirkungsvoll gekühlt wird, ohne dass das Gewebe durch zu tiefe Temperaturen geschädigt wird. Die Kühlwirkung bleibt wegen der grossen Kühlkapazität des Eises über längere Zeit erhalten. Es empfiehlt sich, die Behandlung nach etwa 20 Minuten zu wiederholen, bei schweren Verbrennungen mit zunehmendem zeitlichen Abstand mehrmals. Die beschriebene Behandlung ist eine Erste-Hilfe-Massnahme und ersetzt nicht die Behandlung durch einen Arzt, sichert dieser jedoch erheblich grössere Erfolgsaussichten.

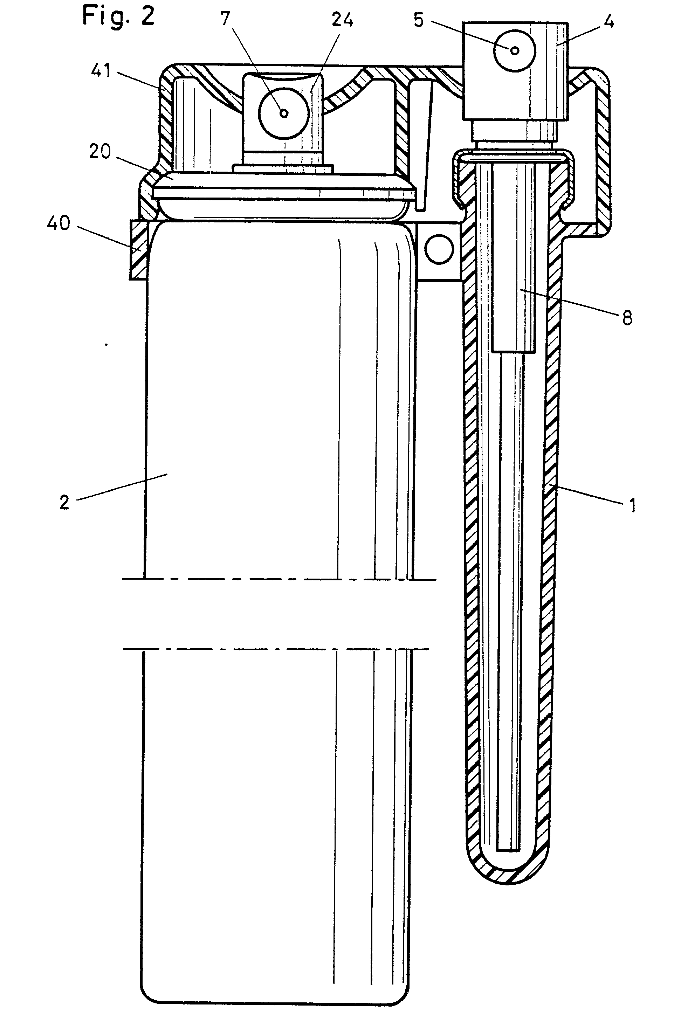

Bei der Ausführungsform nach Fig. 2 sind die beiden Behälter 1, 2 nebeneinander angeordnet. Der Behälter 1 ist Teil eines auf den Behälter 2 aufgesteckten Gehäuses 40, das durch eine auf den Bördelrand 20 des Behälters 2 aufgeschnappte Führungskappe 41 gesichert ist. Die Führungskappe 41 hat Führungsschlitze zum Führen der beiden Sprühdüsen 5, 7, analog dem Führungsschlitz 34 bei der Ausführungsform nach Fig. 1.

Als Kältemittel eignen sich insbesondere fluorhaltige Halogenkohlenwasserstoffe, welche weder brennbar noch giftig sind und keine Reizung der Schleimhäute verursachen. Solche Verbindungen sind als Sicherheitskältemittel bekannt. Besonders geeignet ist Dichlordifluormethan ($CCl_2F_2$) und/oder Chlordifluormethan ($CHClF_2$) mit einem Siedepunkt bei 1 bar von -29,8°C bzw. -40,8°C. Zur Einstellung des Dampfdruckes und damit auch der Kältewirkung kann $CCl_2F_2$ und/oder $CHClF_2$ mit $CCl_3F$, mit $CClF_2 - CCl_2F$ oder mit $CClF_2 - CClF_2$ gemischt werden.

## Patentansprüche

1. Vorrichtung zur Behandlung von Brandwunden oder Verbrühungen, mit zwei aneinander befestigten, getrennten Behältern (1, 2), von denen der erste (1) eine Flüssigkeit (3) enthält und ein Betätigungsglied (4) mit einer Sprühdüse (5) zum Versprühen der Flüssigkeit aufweist, dadurch gekennzeichnet, dass der zweite Behälter (2) ein Kältemittel enthält und ein separates Ventil (6) mit einer zweiten Sprühdüse (7) aufweist, die annähernd parallel zur ersten Sprühdüse (5) ausgerichtet ist.

2. Vorrichtung nach Anspruch 1, dadurch gekennzeichnet, dass der erste Behälter (1) Wasser (3) enthält.

3. Vorrichtung nach Anspruch 1 oder 2, dadurch gekennzeichnet, dass das Betätigungsglied ein Betätigungsknopf (4) einer Pumpe (8) ist.

4. Vorrichtung nach einem der Ansprüche 1 bis 3, dadurch gekennzeichnet, dass das Kältemittel aus fluorhaltigen Halogenkohlenwasserstoffen besteht.

5. Vorrichtung nach einem der Ansprüche 1 bis 4, dadurch gekennzeichnet, dass der erste Behälter (1) koaxial zum zweiten Behälter (2) und über dem Betätigungselement (24) des Ventils (6) angeordnet ist.

6. Vorrichtung nach Anspruch 5, dadurch gekennzeichnet, dass die beiden Behälter (1, 2) durch ein auf den zweiten Behälter (2) aufgeschnapptes Gehäuse (22) miteinander verbunden sind, welches ein Griffloch (25) zum Betätigen des Ventils (6) und diametral gegenüberliegend eine schlitzförmige Öffnung (26) aufweist, in welche die zweite Sprühdüse (7) ragt.

7. Vorrichtung nach Anspruch 6, dadurch gekennzeichnet, dass der erste Behälter (1) im Gehäuse (22) mittels einer aufgeschnappten Führungskappe (27) gehalten ist, die einen Führungsschlitz (34) aufweist, in welchen die erste Sprühdüse (5) ragt, wobei der Führungsschlitz (34) mit der Öffnung (26) fluchtet.

8. Vorrichtung nach einem der Ansprüche 1 bis 4, dadurch gekennzeichnet, dass die beiden Behälter (1, 2) nebeneinander angeordnet sind.

## Claims

1. Apparatus for the treatment of burns or scalds having two separate receptacles (1, 2) fixed to each other, the first (1) thereof containing a liquid (3) and having an operating member (4) with a spray nozzle (5) for the spraying of the liquid, characterized in that, that the second receptacle (2) contains a coolant and has a separate valve (6) with a second spray nozzle (7) which is disposed approximately parallel to the first spray nozzle (5).

2. Apparatus according to claim 1, characterized in that, that the first receptacle (1) contains water (3).

3. Apparatus according to either claim 1 or 2, characterized in that, that the operating member is an operating knob (4) of a pump (8).

4. Apparatus according to any one of claims 1 to 3, characterized in that, that the coolant consists of hydrocarbon halides containing fluorine.

5. Apparatus according to any one of claims 1 to 4, characterized in that, that the first receptacle (1) is disposed coaxially to the second receptacle (2) being positioned above the operating element (24) of the valve (6).

6. Apparatus according to claim 5, characterized in that, that the two receptacles (1, 2) are connected to each other by a housing (22) snapped onto the second receptacle (2), said housing having a grip aperture (25) for activating the valve (6) and diametrically opposite a slot-shaped aperture (26), into which the second spray nozzle (7) projects.

7. Apparatus according to claim 6, characterized in that, that the first receptacle (1) is held in the housing (22) by means of a snapped on guide cap (27) having a guide slit (34), into which the first spray nozzle (5) projects, the guide slit (34) aligning with the aperture (26).

8. Apparatus according to any one of claims 1 to 4, characterized in that, that the two receptacles (1, 2) are disposed side-by-side.

## Revendications

1. Dispositif servant au traitement de brûlures ou d'échaudures et comprenant deux récipients séparés (1, 2) fixés l'un sur l'autre et dont le premier (1) contient un liquide (3) et est pourvu d'un organe de mise en action (4) muni d'une tuyère de projection (5) pour la pulvérisation du

liquide, caractérisé en ce que le deuxième récipient (2) renferme un produit frigorigène et présente une soupape séparée (6) dotée d'une deuxième tuyère de projection (7) alignée presque parallèlement sur la première tuyère de projection (5).

2. Dispositif selon la revendication 1, caractérisé en ce que le premier récipient (1) contient de l'eau (3).

3. Dispositif selon l'une quelconque des revendications 1 et 2, caractérisé en ce que l'organe de mise en action est le bouton de commande (4) d'une pompe (8).

4. Dispositif selon l'une quelconque des revendications 1 à 3, caractérisé en ce que le produit frigorigène se compose d'hydrocarbures halogénés fluorés.

5. Dispositif selon l'une quelconque des revendications 1 à 4, caractérisé en ce que le premier récipient (1) est disposé co-axialement au deuxième récipient (2) et au-dessus de l'élément de mise en action (24) de la soupape (6).

6. Dispositif selon la revendication 5, caractérisé en ce que les deux récipients (1, 2) sont reliés l'un à l'autre par une enveloppe (22) s'emboîtant sur le deuxième récipient (2) et comprenant un creux de maniement (25) pour la mise en action de la soupape (6) et, diamétralement opposée, une ouverture (26) en forme de fente où fait saillie la deuxième tuyère de projection (7).

7. Dispositif selon la revencication 6, caractérisé en ce que le premier récipient (1) est maintenu dans l'enveloppe (22) par un capuchon de guidage emboîté (27) comprenant une fente de guidage (34) où la première tuyère de projection (5) fait saillie, ladite fente de guidage (34) s'alignant sur l'ouverture (26).

8. Dispositif selon l'une quelconque des revendications 1 à 4, caractérisé en ce que les deux récipients (1, 2) sont disposés côte à côte.

## Fig. 1

Fig. 2